(19) Europäisches Patentamt / European Patent Office / Office européen des brevets

(11) **EP 2 966 438 B1**

(12) **FASCICULE DE BREVET EUROPEEN**

(45) Date de publication et mention
de la délivrance du brevet:
**07.11.2018   Bulletin 2018/45**

(51) Int Cl.:
***G01N 27/416*** (2006.01)      ***G01N 33/18*** (2006.01)

(21) Numéro de dépôt: **15173884.6**

(22) Date de dépôt: **25.06.2015**

(54) **PROCÉDÉ ET DISPOSITIF DE MESURE ET D'AFFICHAGE COLORIMÉTRIQUES DE DONNÉES PHYSICO-CHIMIQUES**

VERFAHREN UND VORRICHTUNG ZUR KOLORIMETRISCHEN MESSUNG UND ANZEIGE VON PHYSIKALISCH-CHEMISCHEN DATEN

METHOD AND DEVICE FOR COLOUR GRADING MEASURING AND DISPLAYING OF PHYSICO-CHEMICAL DATA

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **10.07.2014   FR 1456652**

(43) Date de publication de la demande:
**13.01.2016   Bulletin 2016/02**

(73) Titulaire: **Bleu Electrique (SAS)**
**13016 Marseille (FR)**

(72) Inventeur: **BARET, Emmanuel**
**13016 MARSEILLE (FR)**

(74) Mandataire: **Weber, Etienne Nicolas**
**Cabinet Marek**
**28, rue de la Loge**
**Boîte Postale 42413**
**13201 Marseille Cedex 02 (FR)**

(56) Documents cités:
**US-A- 4 033 871      US-A- 4 657 670**
**US-A- 5 218 304**

## Description

La présente invention concerne un procédé de mesure et d'affichage colorimétriques de grandeurs physico-chimiques d'un milieu liquide, et en particulier de l'eau. Elle concerne également un dispositif pour la mise en oeuvre de ce procédé.

Plus particulièrement, la présente invention concerne un procédé et un dispositif de mesure et de signalisation colorimétriques des grandeurs physico-chimiques de l'eau pour la surveillance et l'entretien de l'eau des piscines. Les documents US5218304A et US4033871A divulguent des dispositifs de l'art antérieur.

Pour maintenir la propreté et la limpidité de l'eau des piscines et assurer son bon état sanitaire, il est indispensable de lui appliquer différents traitements. Ces traitements nécessitent de mesurer plusieurs paramètres physico-chimiques entrant en compte dans la définition de l'état de l'eau tels que la température, le potentiel d'hydrogène (PH), le potentiel d'oxydo-réduction (ORP), la conductivité, la salinité ou le taux de solides dissouts, .... Le plus souvent, ces traitements consistent à incorporer des produits chimiques permettant de combattre les causes d'altération de l'eau, de sorte à la rendre propre et transparente. Toutefois, cela implique des vérifications rapprochées de l'état de l'eau par rapport aux différents paramètres physico-chimiques à prendre en compte. D'autre part, cette méthode simple à pour inconvénient de générer une odeur ou de rendre l'eau irritante pour la peau ou pour les yeux, notamment en cours de dosages trop importants des produits de traitement incorporés à l'eau.

Actuellement, il existe de nombreux appareils d'analyse qui proposent un affichage numérique des paramètres physico-chimiques de l'eau en donnant une indication chiffrées des résultats de mesure de ces paramètres. Ces affichages de résultats sont généralement très précis, mais la lecture et l'interprétation de ces résultats sont difficiles et fastidieuses pour les utilisateurs, notamment pour les particuliers.

Un but de la présente invention est de remédier à cet inconvénient, en proposant un dispositif permettant de remplacer l'affichage numérique par un affichage colorimétrique des grandeurs mesurées.

Selon l'invention, ce but est atteint grâce à un dispositif de mesure et de signalisation colorimétriques d'une ou plusieurs grandeurs physico-chimiques d'un milieu liquide, en particulier pour la surveillance et l'entretien de l'eau des piscines. Le dispositif est décrit par la revendication 1.

On entend par mesure colorimétrique une mesure sous la forme d'une indication colorée. L'indication colorée peut prendre différentes formes telles l'émission d'une lumière colorée ou l'éclairage d'un milieu avec une lumière colorée. En particulier le milieu éclairé peut être l'eau dont on souhaite connaître l'état sanitaire ou dont on souhaite connaître les valeurs physico-chimiques. L'indication colorée peut aussi comporter l'affichage d'une ou de plusieurs plages de lecture colorées.

On entend par couleur variable une couleur dont la teinte ou la nuance varie en fonction du signal de commande et donc de la mesure. La couleur fournie par l'indicateur peut provenir d'une ou de plusieurs sources de lumière blanche, associées, par exemple, à des filtres colorés et des commutateurs optiques à cristaux liquides. De préférence cependant, la couleur peut aussi être modifiée, comme décrit plus loin, par la synthèse de couleur de plusieurs sources de lumière, ou par l'utilisation alternée de ces sources.

Le capteur est de préférence un capteur électrochimique sensible à un paramètre physico-chimique du milieu liquide à analyser, ou à la variation d'un paramètre physico-chimique. Bien que l'invention puisse être adaptée au contrôle de différents types de milieux liquides, une application particulière est envisagée, comme indiqué précédemment, pour la surveillance de l'eau, et notamment l'eau des piscines. Dans la suite de la description, il sera ainsi fait référence essentiellement à l'eau. Ainsi, le capteur comprend, par exemple, des électrodes ou une sonde pour la mesure de paramètres tels que le potentiel hydrogyne (PH), une valeur de potentiel d'oxydo réduction, une conductivité, une salinité, un taux de solides dissous, une turbidité, ou une température de l'eau. Le capteur peut comporter en particulier un capteur permettant de mesurer l'intensité d'un courant électrique d'électrolyse tel qu'un ampèremètre.

Ainsi, et toujours à titre d'exemple, la couleur de l'indicateur lumineux peut varier proportionnellement, ou pour le moins en fonction de l'un des paramètres mentionnés ci-dessus. Comme cela apparaît dans la suite de la description, la couleur peut aussi varier en fonction d'une pluralité de paramètres.

Le capteur électrochimique délivre un signal de mesure reflétant la valeur de la grandeur mesurée. Il s'agit, par exemple, d'une tension ou d'un courant de mesure. Celui-ci est fourni au circuit de commande qui le convertit en un ou plusieurs signaux de commande de l'indicateur lumineux.

Selon un mode de mise en oeuvre de l'invention, l'indicateur peut comporter une pluralité de sources lumineuses monochromes, aptes à émettre chacune une lumière avec une couleur propre et avec une intensité variable. Les sources lumineuses monochromes peuvent être agencées de manière à produire ladite couleur variable de l'indicateur lumineux par synthèse de leurs couleurs respectives.

On entend par sources monochromes des sources émettant une lumière avec une couleur déterminée qui leur est propre. Ceci ne préjuge pas de la largeur de leur spectre d'émission, qui toutefois ne représente qu'une portion du spectre visible suffisamment restreinte pour apparaître comme coloré.

De préférence, les sources lumineuses individuelles peuvent être choisies pour émettre dans des couleurs complémentaires, de manière à étendre la palette des couleurs susceptible d'être restituée par l'indication lumineuse résultante. Par exemple, les couleurs peuvent être le rouge, le vert et le bleu. Ceci permet d'utiliser avantageusement des diodes électroluminescentes (LED), facilement disponibles dans ces couleurs, comme sources individuelles monochromes. Comme indiqué ci-dessus, les sources lumineuses individuelles, par exemple les diodes LED peuvent être agencées de manière à réaliser une synthèse lumineuse. Ainsi les diodes sont agencées, par exemple pour éclairer une plage de lecture commune, pour éclairer une zone commune d'un milieu, ou encore pour que leur lumière partage un chemin optique commun.

A la place de plusieurs diodes LED de couleurs différentes, l'indicateur peut aussi comporter une ou plusieurs diodes LED multicolores.

Les diodes LED multicolores, à commande multiple, permettent en effet d'émettre directement une lumière colorée de couleur variable. La synthèse lumineuse a lieu dans ce cas directement à l'intérieur du boîtier de la diode.

Selon la configuration de l'indicateur lumineux, le circuit de commande peut délivrer un ou plusieurs signaux de commande, par exemple en courant ou en tension. Toutefois selon un mode de réalisation de l'invention utilisant une pluralité de sources lumineuses monochromes, le circuit de commande peut être conçu pour produire un ou plusieurs signaux à largeur d'impulsion modulé, respectivement pour chaque source de lumière monochrome, de manière à en ajuster l'intensité lumineuse.

Ainsi, l'ajustage individuel de l'intensité de chaque source permet de contrôler la contribution de la couleur correspondante dans la lumière résultante émise par l'indicateur.

La couleur de l'indicateur lumineux peut être modifiée en fonction d'un unique paramètre et indiquer ainsi une valeur de ce paramètre. Elle peut aussi être modifiée en fonction de plusieurs paramètres et indiquer un état global dela qualité ou de l'état sanitaire de l'eau.

Ainsi, selon un mode de mise en oeuvre de l'invention, le dispositif peut comporter une pluralité de capteurs, aptes à mesurer respectivement des paramètres physico-chimiques distincts du milieu liquide ou de l'eau à analyser. Le circuit de commande est relié respectivement à chaque capteur et conçu pour produire un signal de commande distinct respectivement pour chacune des sources lumineuse monochromes de l'indicateur, en réponse respectivement à la mesure d'un capteur distinct, de telle manière que l'intensité lumineuse émise par chaque source monochrome est fonction respectivement de la mesure d'un capteur.

Lorsque plusieurs grandeurs sont mesurées, une couleur primaire distincte peut être attribuée à chacune des grandeurs physico-chimiques de sorte que la couleur résultante donne une indication instantanée et intuitive de la valeur des grandeurs correspondantes.

Les composants du système de mesure et d'affichage, en particulier le ou les capteurs et l'indicateur lumineux peuvent être logés dans un boîtier étanche. Ce boîtier comprend, par exemple, un corps creux délimitant une chambre de mesure et un couvercle. Il peut être muni d'une ouverture d'entrée et d'une ouverture de sortie, permettant une circulation du milieu liquide, et en particulier de l'eau à l'intérieur dudit boîtier. Par ailleurs, au moins un élément ou une partie de ce boîtier peut être réalisé dans un matériau transparent ou translucide.

Le boîtier peut en outre être muni d'une ouverture d'entrée (5) et d'une ouverture de sortie (6) destinées à un branchement du dispositif sur une conduite (C) de sortie d'une piscine, de sorte que, lorsque le boitier (4) est installé sur la conduite, le ou les capteurs se trouvent immergés dans un courant d'eau traversant le boîtier.

L'invention concerne également un procédé de mesure et d'affichage colorimétriques de grandeurs physico-chimiques d'un milieu liquide selon la revendication 11 et en particulier de l'eau. Selon le procédé, on immerge dans l'eau dont la salubrité ou l'état sanitaire doit être analysé, au moins un capteur sensible à au moins un paramètre physico-chimique, en particulier un paramètre contribuant à la limpidité de l'eau analysée et/ou au bon état sanitaire de celle-ci, on fournit au moins un signal de mesure du capteur à un circuit de commande d'un indicateur lumineux apte à émettre une lumière de couleur variable, et on fournit un signal de commande électrique du circuit de commande à l'indicateur de manière à provoquer l'émission d'une lumière dont la couleur est fonction de la mesure du ou des capteurs.

Le capteur peut être en particulier un capteur électrochimique apte à mesurer les paramètres mentionnés précédemment.

Selon une mise en oeuvre du procédé on peut utiliser un indicateur lumineux avec une pluralité de sources lumineuses monochromes complémentaires, aptes à émettre chacune une lumière avec une couleur propre, avec une intensité variable, les sources lumineuses monochromes étant agencées de manière à produire ladite couleur variable par synthèse des couleurs.

Par ailleurs, et toujours selon une mise en oeuvre particulière du procédé, on peut utiliser la lumière émise par l'indicateur lumineux pour éclairer le milieu dont la salubrité ou l'état sanitaire soit être analysé. Selon cette mise en oeuvre, l'eau ou le milieu analysé devient lui-même l'afficheur de ses propres caractéristiques.

Le procédé et le dispositif selon l'invention procurent plusieurs avantages intéressants, notamment pour le contrôle de l'eau des piscines. Il s'agit, en l'occurrence :

- de permettre une lecture rapide et simple des paramètres physico-chimiques auxquels doit répondre une eau limpide et de bonne qualité sanitaire,

- de permettre un contrôle instantané et simultané de plusieurs paramètres physico-chimiques de l'eau analysée.

Les buts, caractéristiques et avantages ci-dessus, et d'autres encore, ressortiront mieux de la description détaillée qui suit et des dessins annexés dans lesquels :

La figure 1 est une vue à caractère schématique illustrant le dispositif de l'invention pour la mesure et l'affichage d'une seule grandeur physico-chimique.

La figure 2 est une vue à caractère schématique illustrant le dispositif de l'invention pour la mesure et l'affichage de plusieurs grandeurs physico-chimiques et, plus précisément, de trois grandeurs physico-chimiques d'une eau à analyser.

La figure 3 est une vue à caractère schématique illustrant un mode d'exécution du dispositif de l'invention suivant lequel les capteurs électrochimiques sont constitués par les électrodes d'un électrolyseur.

La figure 4 est une vue à caractère schématique illustrant le dispositif de l'invention pour la mesure et l'affichage du PH.

La figure 5 représente le schéma d'affichage du PH en fonction de trois LEDs, Rouge, Verte, Bleue.

On se réfère auxdits dessins pour décrire des exemples intéressants quoique nullement limitatifs, de réalisation du dispositif de mesure et d'affichage colorimétriques et de mise en oeuvre du procédé selon l'invention.

Selon ce procédé, on immerge, dans l'eau E à analyser, au moins un capteur électrochimique 1 conçu pour être sensible à, au moins l'un des paramètres physico-chimiques nécessaires au maintien des qualités que doit présenter l'eau dans certaines destinations de celle-ci, ou aux variations de ce paramètre.

Plus particulièrement, dans l'application au maintien de la limpidité et des qualités hygiéniques de l'eau des piscines, cette eau est définie par une pluralité de paramètres physico-chimiques tels que :

- la température ;

- le PH ;

- le potentiel d'oxydoréduction (ORP) ;

- la salinité ou le taux de solides dissouts ;

- la conductivité de l'eau.

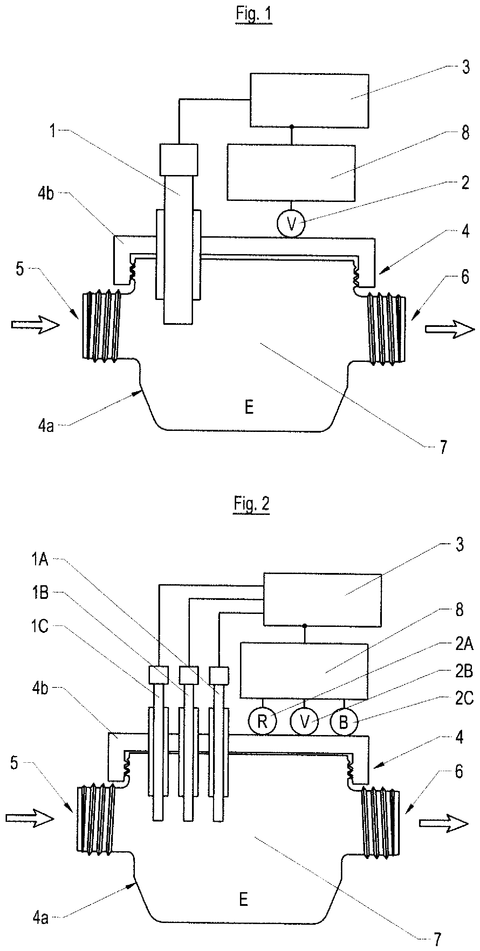

On connecte électriquement ce capteur électrochimique 1 à un indicateur lumineux 2 conçu pour émettre une lumière colorée qui varie en fonction de la valeur de la grandeur mesurée par ledit capteur (figure 1).

De préférence, on immerge (figure 2), dans l'eau E à analyser, une pluralité de capteurs électrochimiques 1A, 1B, 1C, ... conçus, chacun, pour être sensibles à l'un des paramètres physico-chimiques spécifiques à prendre en considération pour la définition d'une eau saine. Chacun de ces capteurs électrochimiques 1A, 1B, 1C, ... est dédié à la mesure de l'état de l'un des paramètres physico-chimiques de l'eau.

Chaque capteur électrochimique 1A, 1B, 1C, ... est connecté électriquement à au moins une source de lumière colorée 2A, 2B, 2C émettant une lumière dans une couleur qui lui est propre, de sorte que l'intensité de la lumière colorée de chaque source lumineuse varie en fonction de la valeur de la grandeur physico-chimique mesurée (détectée) par le capteur électrochimique qui lui est associé.

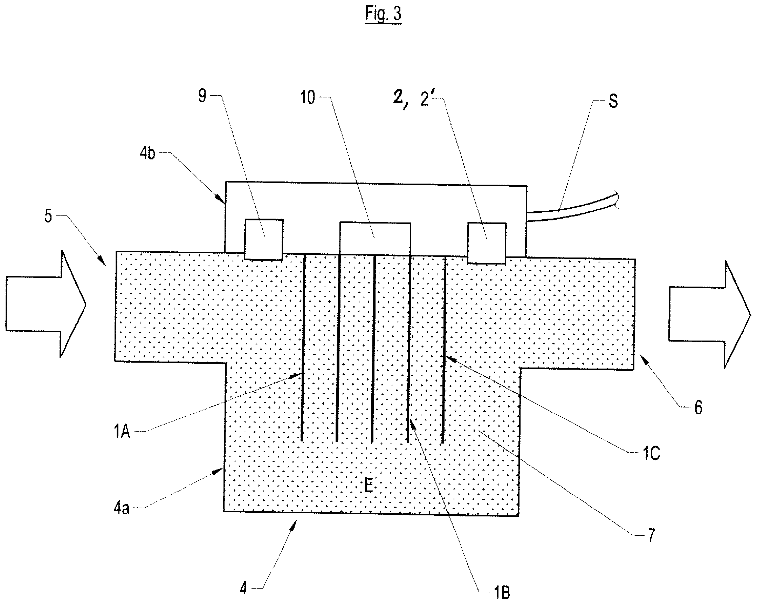

Selon un autre mode de mise en oeuvre du procédé de l'invention, l'ensemble des capteurs électrochimiques 1A, 1B, 1C, ... est connecté à une source de lumière multicolore 2' (figure 3).

Selon le procédé de l'invention, on attribue une source lumineuse 2A, 2B, 2C, d'une couleur déterminée respectivement à une grandeur physico-chimique mesurée par un capteur électro-chimique 1A, 1B, 1C, ... telle que la température, le potentiel d'hydrogène (PH), le potentiel d'oxydoréduction (ORP), ..., de sorte que l'intensité lumineuse de ladite source lumineuse varie respectivement en fonction de la valeur de la grandeur physico-chimique mesurée.

Selon un exemple de mise en oeuvre avantageux du procédé de l'invention dans lequel plusieurs grandeurs sont mesurées, des couleurs complémentaires, par exemple des couleurs primaires, sont attribuées à chacune des grandeurs physico-chimiques de sorte que la couleur résultante donne une indication instantanée et intuitive de la valeur des grandeurs correspondantes.

Dans sa version la plus simple, le dispositif d'analyse et d'affichage selon l'invention comprend notamment :

- au moins un capteur électrochimique 1 conçu pour être sensible à au moins un paramètre physico-chimique contribuant à la limpidité de l'eau analysée et au bon état sanitaire de celle-ci ; ce capteur électrochimique étant connecté électriquement :

  - d'une part, à une source d'alimentation électrique qui peut être le courant du secteur ou une batterie, et dont on a seulement représenté le câble d'alimentation S sur la figure 3, et

  - d'autre part, à au moins une source de lumière colorée de l'indicateur 2, conçue pour émettre une lumière colorée ;

- le circuit électronique de commande reliant le capteur électrochimique à la source de lumière colorée 2,

Le capteur électrochimique 1, l'indicateur lumineux 2 et le circuit électronique 3 constituent, ensemble, une unité de mesure et d'affichage des grandeurs mesurées.

Les éléments actifs de cette cellule de mesure et d'affichage sont logés dans un boîtier étanche 4, ce boitier étant, par exemple, constitué d'un corps creux 4a et d'un couvercle ou capot 4b, et muni d'une ouverture d'entrée 5 et d'une ouverture de sortie 6 permettant une circulation de l'eau à l'intérieur dudit boitier. Au moins un élément ou partie constitutive du boitier est réalisé(e) dans un matériau transparent ou translucide, de sorte que la lumière émise par la ou les sources lumineuses est visible à travers la partie transparente du boîtier.

Le capot ou couvercle 4b est, par exemple, fixé par vissage sur le corps 4a du boîtier 4. Dans le cas de l'application à l'assainissement de l'eau des piscines, le boîtier 4 est placé dans le local technique, sur le circuit de filtration, en aval de l'orifice de sortie 6 de l'eau de la piscine sur une conduite de retour C.

Le boîtier 4 délimite, intérieurement, une chambre de mesure 7 traversée par le courant ou flux d'eau circulant dans la conduite C et dans laquelle sont immergés le ou les capteurs électrochimiques 1.

De préférence, plusieurs capteurs électrochimiques 1A, 1B, 1C, ... sont conçus, chacun, pour être sensible à au moins un paramètre physico-chimique spécifique de l'eau à analyser et associé à au moins une source de lumière colorée émettant dans une couleur différente de celle des autres sources de lumière. Ainsi, le dispositif comprend une pluralité d'unités de détection comprenant, chacune, un capteur électrochimique 1 et une source de lumière colorée 2 connectée à ce dernier par l'intermédiaire du circuit de commande.

Selon une variante, le ou les capteurs sont reliés à une source de lumière multicolore 2' conçue pour émettre une lumière dans une couleur prédéterminée dédiée à l'affichage de la grandeur mesurée de l'un des paramètres physico-chimiques.

De préférence, l'unité de détection 1-2-3 ou chaque unité de détection 1-2-3 est intercalée dans une canalisation de sortie de la piscine, par tout moyen de raccordement étanche approprié, de sorte à permettre une circulation de l'eau de la piscine.

De préférence et avantageusement, le couvercle ou capot 4b du boîtier 4 est exécuté dans un matériau transparent ou translucide afin de permettre la lecture de l'affichage colorimétrique résultant de l'émission de la ou des sources lumineuses 2A, 2B, 2C, ..., le corps 4a dudit boîtier étant réalisé dans un matériau opaque.

Le couvercle ou capot 4b est fixé, par exemple par vissage, sur l'extrémité supérieure filetée du corps 4a du boîtier 4.

Le ou les capteurs 1A, 1B, 1C, ..., et la ou les sources lumineuses 2A, 2B, 2C, ..., sont fixés sur le capot 4b du boîtier 4, de sorte que lorsque le capot est fermé, le ou les capteurs est/sont immergé(s) dans l'eau traversant la chambre de mesure 7, ménagée dans ledit boîtier, et les sources lumineuses 2A, 2B, 2C, ..., émettent en direction de ladite chambre.

Le dispositif de détection et d'affichage, et plus précisément le circuit électronique de commande 3, comprend encore un dispositif de contrôle 8 connu en soi ou à la portée de l'homme du métier. Ce dispositif de contrôle est dans cet exemple un circuit électronique dédié au pilotage des LEDs. Il permet de contrôler l'intensité des LEDs Rouges, Vertes et Bleues de façon à composer la nuance de la couleur à afficher selon les informations de mesure reçues. Le dispositif de contrôle 8 permet la production d'un signal à largeur d'impulsion modulé pour chaque couleur primaire, de sorte à en ajuster l'intensité lumineuse.

Suivant une autre disposition, le dispositif d'affichage comprend encore un capteur de température permettant de détecter la température de l'eau analysée.

Avantageusement, pour la lecture de plusieurs grandeurs physico-chimiques, le dispositif comporte plusieurs capteurs 1A, 1B, 1C, ..., pour mesurer différentes grandeurs physico-chimiques, chacun de ces capteurs étant relié à une source de lumière, par exemple constituée par une LED, avec laquelle il forme une unité de détection et d'affichage colorimétrique. La ou les LEDs 2' ; 2A, 2B, 2C, ..., sont conçues pour émettre une lumière dans l'une des couleurs primaires (vert, rouge, bleu) ou dans des nuances de ces couleurs.

Selon un autre exemple de réalisation pour la lecture de plusieurs grandeurs physico-chimiques, les différents capteurs 1A, 1B, 1C, ..., sont reliés à une seule source de lumière 2', par exemple constituée par une LED multicolore.

Comme indiqué précédemment, le circuit électronique de commande 3, est configuré pour faire varier l'intensité lumineuse de la ou des LEDs en fonction des valeurs de la ou des grandeur(s) physico-chimique(s) mesurées par le ou les capteurs 1A, 1B, 1C, ....

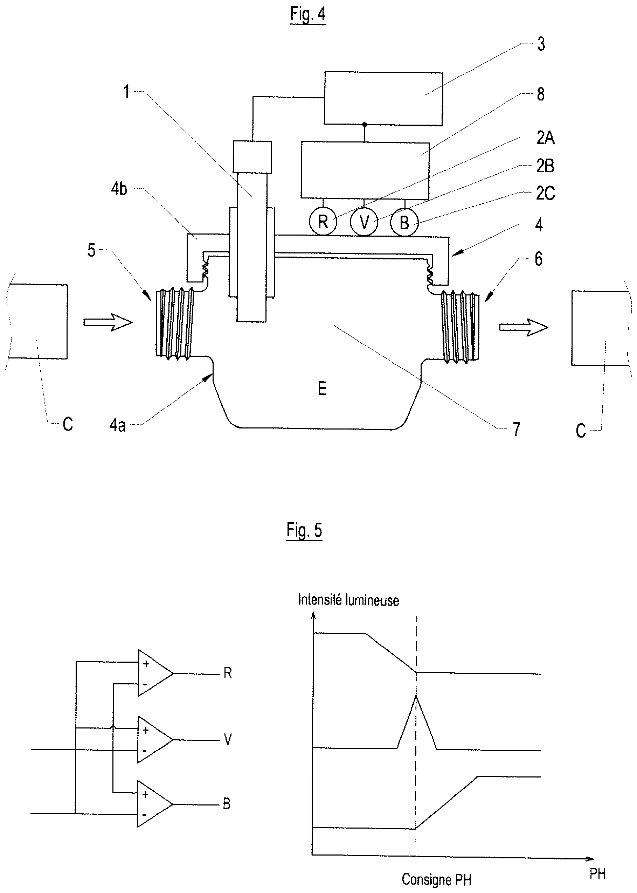

Selon l'exemple de réalisation illustré à la figure 4, le dispositif de détection et d'affichage comprend une sonde ou capteur 1 apte à mesurer le PH de l'eau E circulant dans le boîtier 4. Selon le mode d'exécution illustré à la figure 3, les capteurs électrochimiques 1A, 1B, 1C, ..., sont constitués par des électrodes d'un dispositif d'électrolyse ou électrolyseur, immergées dans un flux d'eau traversant une chambre de mesure électrolytique 7 ou cellule, et traversées par un courant électrique continu d'électrolyse.

Comme illustré à la figure 5, pour la mesure et l'affichage du potentiel d'hydrogène (PH), on attribue, par exemple :

- une LED de couleur verte à cette grandeur, lorsqu'elle est proche de sa valeur de consigne, c'est-à-dire lorsqu'elle est proche de 7,2,

- une LED de couleur rouge lorsque le PH est inférieur à cette valeur de consigne et,

- une LED de couleur bleue lorsque le PH est supérieur à cette valeur de consigne.

Ainsi, lorsque le PH de l'eau analysée diminue, l'intensité lumineuse produite par la LED rouge augmente tandis que les intensités lumineuses produites par les LEDs verte et bleue diminuent, de sorte que la cellule est éclairée en rouge, indiquant que le PH est inférieur à la valeur souhaitée.

Lorsque le PH est proche de sa valeur de consigne, l'intensité lumineuse produite par la LED verte augmente, tandis que les intensités lumineuses produites par les LEDs rouge et bleue diminuent, de sorte que la cellule est éclairée en vert, indiquant que le PH est idéal.

Lorsque le PH augmente, l'intensité lumineuse produite par la LED bleue augmente, tandis que les intensités lumineuses produites par les LEDs rouge et verte diminuent, de sorte que la cellule est éclairée en bleu, indiquant que le PH est supérieur à la valeur souhaitée.

Selon un autre exemple de réalisation pour la mesure et l'affichage du potentiel d'oxydoréductions (ORP), on attribue, par exemple, une LED de couleur verte à cette grandeur. Ainsi, lorsque la valeur mesurée s'écarte de la valeur de consigne souhaitée, par exemple égale à 650 mV, l'intensité lumineuse produite par la LED verte augmente, de sorte que la cellule est éclairée en vert.

Selon un autre exemple de réalisation, dans lequel l'utilisateur souhaite surveiller à la fois le potentiel d'hydrogène (PH) et le potentiel d'oxydoréduction (ORP), on attribue, par exemple, une LED de couleur rouge à la mesure du PH et une LED de couleur verte à la mesure de l'ORP, de sorte que :

- si la valeur du PH s'écarte de la valeur de consigne et que la valeur de l'ORP demeure proche de la valeur de consigne, seule l'intensité de la LED rouge augmente et la cellule est éclairée en rouge ;

- si la valeur de l'ORP s'écarte de la valeur de consigne et que la valeur du PH demeure proche de la valeur de consigne, seule l'intensité de la LED verte augmente et la cellule est éclairée en vert ;

- si la valeur du PH et celle de l'ORP s'écartent simultanément de leur valeur de consigne respective, alors l'intensité lumineuse des deux LEDs rouge et verte augmente de sorte que la cellule est éclairée en jaune.

Ainsi, l'observation de la seule couleur résultante permet de surveiller plusieurs grandeurs physico-chimiques.

De nombreuses piscines sont équipées d'électrolyseurs qui produisent du chlore par électrolyse de l'eau salée. Ces dispositifs sont généralement constitués d'électrodes qui, malgré un traitement approprié, s'oxydent, ce qui réduit leur durée de vie. Ainsi, pour optimiser la durée de vie d'une cellule d'électrolyse, il convient de maintenir le courant d'électrolyse dans une plage déterminée, comprise entre une intensité de courant minimum **Imin** et une intensité de courant maximum **Imax.** Or, le courant d'électrolyse dépend de la conductivité de l'eau qui elle-même dépend de la température et de la salinité de l'eau.

Ainsi, pour assurer le bon fonctionnement de ces dispositifs d'électrolyse, l'utilisateur doit surveiller l'intensité du courant d'électrolyse le et agir, si besoin, sur la salinité de l'eau de la piscine.

Selon un exemple de mise en oeuvre, spécifique à la surveillance du courant d'électrolyse :

- une LED rouge est attribuée à une production faible de courant d'électrolyse ;

- une LED bleue est attribuée à une production importante de courant d'électrolyse ;

- un capteur mesure l'intensité du courant d'électrolyse ;

- la puissance lumineuse de la LED bleue (**Pbleu**) est modulée selon la formule :

$$Pbleu = (Imax - Ie) / (Imax - Imin) \times 100\%$$

- la puissance lumineuse de la LED rouge (**Prouge**) est modulée selon la formule :

$$Prouge = (Ie - Imin) / (Imax - Imin) \times 100\%$$

De la sorte :

- si la production de courant d'électrolyse Ie est trop faible, la cellule est éclairée en rouge ;

- si la production de courant d'électrolyse Ie est trop élevée, la cellule est éclairée en bleu ;

- si la production de courant d'électrolyse Ie est idéale, la cellule est éclairée en magenta (couleur résultante de l'éclairage des LEDs bleue et rouge).

Ainsi, la lecture et l'interprétation de l'information est simple, et l'utilisateur peut facilement corriger la salinité de l'eau.

La modulation de la puissance peut avoir lieu en faisant varier un courant de commande ou en faisant varier un rapport cyclique d'allumage de la diode électroluminescente correspondante. Par exemple, on peut faire varier une largeur d'impulsions de courant d'alimentation.

La cellule peut encore contenir un capteur de température 9 du flux d'eau traversant la chambre de mesure 7 (figure 3). Les données issues de ce capteur sont alors liées à la puissance lumineuse d'une LED, par exemple de couleur verte. En outre, sur la figure 3, la référence 10 désigne un capteur de courant. Ce capteur de courant 10 permet de mesurer l'intensité du courant électrique continu d'électrolyse.

Selon un exemple de réalisation dans lequel on surveille à la fois la température et la conductivité de l'eau, la température peut être affichée à l'aide d'une LED verte, et la conductivité de l'eau peut être affichée à l'aide de LEDs rouge et bleue. Lorsque l'électrolyse est à l'arrêt, seul le capteur de température est actif et seule la LED verte s'éclaire de sorte que la cellule est éclairée en fonction de la température de l'eau uniquement. Lorsque l'électrolyse est en production, la couleur de l'éclairage de la cellule varie, à la fois en fonction de l'éclairage de la LED verte indiquant la température de l'eau, mais également en fonction l'éclairage des LEDs rouge et bleue de la conductivité de l'eau (rouge, bleu ou magenta en fonction de la production de courant d'électrolyse).

## Revendications

1. Dispositif de mesure et de signalisation colorimétriques d'une ou plusieurs grandeurs physico-chimiques d'un milieu liquide, comprenant :

   - au moins un capteur (1) apte à mesurer au moins un paramètre physico-chimique du milieu ;
   - au moins un indicateur lumineux (2) conçu pour émettre une lumière colorée avec une couleur variable en fonction d'un signal électrique de commande, l'indicateur lumineux (2) comprenant une pluralité de sources lumineuses monochromes (2A, 2B, 2C), aptes à émettre, chacune, une lumière avec une couleur propre, et avec une intensité variable, les sources lumineuses monochromes étant agencées de manière à produire ladite couleur variable par synthèse des couleurs propres des sources lumineuses monochromes ;
   - un circuit électronique de commande (3) relié au capteur (1) et configuré pour convertir au moins une mesure du capteur en un signal électrique de pilotage de l'indicateur pour provoquer l'émission d'une lumière avec une couleur résultante fonction de la mesure de dudit paramètre physico-chimique.

**2.** Dispositif de mesure et de signalisation colorimétriques selon la revendication 1, dans lequel le circuit de commande est adapté à produire un signal à largeur d'impulsion modulé pour chaque source de lumière monochrome, de sorte à en ajuster l'intensité lumineuse.

**3.** Dispositif de mesure et de signalisation colorimétriques selon l'une des revendications 1 et 2, **caractérisé en ce qu'**il comprend une pluralité de capteurs (1A, 1B, 1C, ...) aptes à mesurer respectivement des paramètres physico-chimiques distincts du milieu à analyser, le circuit de commande étant relié respectivement à chaque capteur et étant apte à produire un signal de pilotage distinct respectivement pour chacune des sources lumineuses (2A, 2B, 2C) de l'indicateur (2), en réponse respectivement à la mesure d'un capteur distinct (1A, 1B, 1C), de telle manière que l'intensité lumineuse émise par chaque source est fonction respectivement de la mesure d'un capteur.

**4.** Dispositif de mesure et de signalisation colorimétriques selon la revendication 1, comprenant en outre une chambre de mesure électrolytique (7), et dans lequel le capteur (1A, 1B, 1C, ...) comporte des électrodes susceptibles d'être immergées dans un flux d'eau traversant la chambre de mesure électrolytique.

**5.** Dispositif de mesure et de signalisation colorimétriques selon la revendication 1, comprenant un boîtier (4) avec un corps creux (4a) et un couvercle (4b) lesquels délimitent une chambre de mesure étanche (7), au moins une partie du boitier étant réalisée dans un matériau transparent ou translucide, le capteur (1,1A, 1B, 1C, ...) et l'indicateur lumineux étant logés dans le boîtier (4).

**6.** Dispositif de mesure et de signalisation colorimétriques selon la revendication 5, dans lequel le couvercle (4b) du boîtier est réalisé dans un matériau transparent ou translucide, et dans lequel l'indicateur lumineux (2, 2A, 2B, 2C, ...) est logé dans le couvercle (4b) du boîtier (4).

**7.** Dispositif de mesure et de signalisation colorimétriques selon la revendication 5, dans lequel le boîtier (4) est muni d'une ouverture d'entrée (5) et d'une ouverture de sortie (6) destinées à permettre le branchement dudit dispositif sur une conduite (C) de sortie d'une piscine, de sorte que lorsque le boîtier (4) est installé sur ladite conduite, le capteur (1 ; 1A, 1B, 1C) se trouve immergé dans un courant d'eau traversant le boîtier.

**8.** Dispositif de mesure et de signalisation colorimétriques selon la revendication 1, comprenant un capteur (10) permettant de mesurer l'intensité d'un courant électrique d'électrolyse et un capteur de température (9).

**9.** Dispositif de mesure et de signalisation colorimétrique selon la revendication 1, dans lequel l'indicateur lumineux (2) comporte au moins une diode électroluminescente multicolore (2').

**10.** Dispositif de mesure et de signalisation colorimétrique selon la revendication 1, dans lequel les sources monochromes (2A, 2B, 2C) comportent des diodes électroluminescentes (LED) de couleurs complémentaires.

**11.** Procédé de mesure et d'affichage colorimétriques de grandeurs physico-chimiques d'un milieu liquide, **caractérisé en ce que** l'on immerge, dans le milieu, au moins un capteur (1) sensible à au moins un paramètre physico-chimique, on fournit au moins un signal de mesure du capteur à un circuit de commande (3) d'un indicateur lumineux (2) apte à émettre une lumière de couleur variable, et on fournit un signal de commande du circuit de commande à l'indicateur lumineux de manière à émettre une lumière dont la couleur est fonction de la mesure du capteur (1), dans lequel on utilise ledit indicateur lumineux (2) avec une pluralité de sources lumineuses (2A, 2B, 2C) monochromes, aptes à émettre chacune une lumière avec une couleur propre, et avec une intensité variable, les sources lumineuses monochromes étant agencées de manière à produire ladite couleur variable par synthèse des couleurs propres.

**12.** Procédé de mesure et d'affichage colorimétriques selon la revendication 11, dans lequel on utilise une pluralité de capteurs (1A, 1B, 1C, ...) distincts conçus, chacun, pour être sensible à l'un des paramètres physico-chimiques du milieu, et on fournit un signal de commande électrique à chaque source lumineuse (2A 2B, 2C) respectivement en fonction d'un signal de mesure de chaque capteur, de manière que chaque source lumineuse de l'indicateur (2) émette une lumière avec une intensité fonction de la mesure du capteur correspondant.

**13.** Procédé selon la revendication 11, dans lequel le milieu liquide est de l'eau dont la salubrité ou l'état sanitaire doit être analysé, et dans lequel on utilise la lumière émise par l'indicateur lumineux pour éclairer le milieu.

**Patentansprüche**

1. Vorrichtung zur kolometrischen Messung und Signalisierung einer oder mehrerer physikalisch-chemischer Größen eines flüssigen Mediums, umfassend:

   - zumindest einen Sensor (1), der dazu in der Lage ist, zumindest einen physikalisch-chemischen Parameter des Mediums zu messen;
   - zumindest eine Leuchtanzeige (2), die dazu ausgelegt ist, ein farbiges Licht mit einer variablen Farbe in Abhängigkeit von einem elektrischen Steuersignal zu emittieren, wobei die Leuchtanzeige (2) eine Vielzahl von monochromen Lichtquellen (2A, 2B, 2C) umfasst, die dazu in der Lage sind, jeweils ein Licht mit einer eigenen Farbe und mit einer variablen Intensität zu emittieren, wobei die monochromen Lichtquellen angeordnet sind, um die variable Farbe durch Synthese der eigenen Farben der monochromen Lichtquellen zu erzeugen;
   - eine elektronische Steuerschaltung (3), die mit dem Sensor (1) verbunden und ausgelegt ist, um zumindest eine Messung des Sensors in ein elektrisches Steuersignal der Anzeige umzuwandeln, um die Emission von Licht mit einer resultierenden Farbe in Abhängigkeit von der Messung des physikalisch-chemischen Parameters zu bewirken.

2. Vorrichtung zur kolometrischen Messung und Signalisierung nach Anspruch 1, wobei die Steuerschaltung ausgelegt ist, um ein impulsbreitenmoduliertes Signal für jede monochrome Lichtquelle zu erzeugen, um die Lichtintensität einzustellen.

3. Vorrichtung zur kolometrischen Messung und Signalisierung nach einem der Ansprüche 1 und 2, **dadurch gekennzeichnet, dass** sie eine Vielzahl von Sensoren (1A, 1B, 1C,...) umfasst, die jeweils dazu in der Lage sind, einzelne physikalischchemische Parameter des zu analysierenden Mediums zu messen, wobei die Steuerschaltung jeweils mit jedem Sensor verbunden und dazu in der Lage ist, jeweils ein separates Steuersignal für jede der Lichtquellen (2A, 2B, 2C) der Anzeige (2) zu erzeugen, als Reaktion auf die jeweilige Messung eines separaten Sensors (1A, 1B, 1C), sodass die von jeder Quelle emittierte Lichtintensität jeweils von der Messung eines Sensors abhängig ist.

4. Vorrichtung zur kolometrischen Messung und Signalisierung nach Anspruch 1, femer umfassend eine elektrolytische Messkammer (7), und wobei der Sensor (1A, 1B, 1C,...) Elektroden umfasst, die in einen Wasserfluss durch die elektrolytische Messkammer eingetaucht werden können.

5. Vorrichtung zur kolometrischen Messung und Signalisierung nach Anspruch 1, umfassend ein Gehäuse (4) mit einem Hohlkörper (4a) und einem Deckel (4b), die eine abgedichtete Messkammer (7) begrenzen, wobei zumindest ein Abschnitt des Gehäuses aus einem transparenten oder lichtdurchlässigen Material gefertigt ist, wobei der Sensor (1, 1A, 1B, 1C,...) und die Leuchtanzeige in dem Gehäuse (4) untergebracht sind.

6. Vorrichtung zur kolometrischen Messung und Signalisierung nach Anspruch 5, wobei der Deckel (4b) des Gehäuses aus einem transparenten oder lichtdurchlässigen Material gefertigt ist, und wobei die Leuchtanzeige (2, 2A, 2B, 2C,...) in dem Deckel (4b) des Gehäuses (4) untergebracht ist.

7. Vorrichtung zur kolometrischen Messung und Signalisierung nach Anspruch 5, wobei das Gehäuse (4) mit einer Einlassöffnung (5) und einer Auslassöffnung (6) bereitgestellt ist, um den Anschluss der Vorrichtung an eine Auslassleitung (C) aus einem Becken zu ermöglichen, sodass, wenn das Gehäuse (4) an der Leitung angebracht ist, der Sensor (1; 1A, 1B, 1C) in einen Wasserstrom durch das Gehäuse eingetaucht ist.

8. Vorrichtung zur kolometrischen Messung und Signalisierung nach Anspruch 1, umfassend einen Sensor (10) zum Messen der Intensität eines elektrischen Stroms der Elektrolyse und einen Temperatursensor (9).

9. Vorrichtung zur kolometrischen Messung und Signalisierung nach Anspruch 1, wobei die Leuchtanzeige (2) zumindest eine mehrfarbige Leuchtdiode (2') umfasst.

10. Vorrichtung zur kolometrischen Messung und Signalisierung nach Anspruch 1, wobei die monochromen Quellen (2A, 2B, 2C) Leuchtdioden (LED) komplementärer Farben umfassen.

11. Verfahren zur kolometrischen Messung und Anzeige physikalisch-chemischer Größen eines flüssigen Mediums, **dadurch gekennzeichnet, dass** in das Medium zumindest ein Sensor (1) eingetaucht wird, der zumindest auf einen physikalisch-chemischen Parameter reagiert, zumindest ein Messsignal des Sensors einer Steuerschaltung (3) einer Leuchtanzeige (2) bereitgestellt wird, die dazu in der Lage ist, Licht einer variablen Farbe zu emittieren, und der Leuchtanzeige ein Steuersignal der Steuerschaltung bereitgestellt wird, um Licht zu emittieren, dessen Farbe von der Messung des Sensors (1) abhängt, wobei die Leuchtanzeige (2) mit einer Vielzahl von monochromen Lichtquellen (2A, 2B, 2C) verwendet wird, die jeweils dazu in der Lage sind, ein Licht in einer eigenen Farbe und mit einer variablen Intensität zu emittieren, wobei die monochromen Lichtquellen angeordnet sind, um die variable Farbe durch Synthese eigener Farben zu erzeugen.

12. Verfahren zur kolometrischen Messung und Anzeige nach Anspruch 11, wobei eine Vielzahl von separat gestalteten Sensoren (1A, 1B, 1C,...) verwendet wird, die jeweils auf einen der physikalisch-chemischen Parameter des Mediums reagieren, und jeder Lichtquelle (2A, 2B, 2C) ein elektrisches Steuersignal jeweils in Abhängigkeit eines Messsignals jedes Sensors bereitgestellt wird, sodass jede Lichtquelle der Anzeige (2) ein Licht mit einer Intensität in Abhängigkeit von der Messung des entsprechenden Sensors emittiert.

13. Verfahren nach Anspruch 11, wobei das flüssige Medium Wasser ist, dessen Unbedenklichkeit oder Gesundheitszustand zu analysieren ist, und wobei das von der Leuchtanzeige emittierte Licht verwendet wird, um das Medium zu beleuchten.

## Claims

1. A device for colorimetric measurement and signalling of one or more physico-chemical values of a liquid medium, comprising:

   - at least one sensor (1) capable of measuring at least one physico-chemical parameter of the medium;
   - at least one light indicator (2) designed to emit a coloured light of a variable colour in dependence on an electrical control signal, the light indicator (2) comprising a plurality of monochrome light sources (2A, 2B, 2C) capable of each emitting a light of a specific colour and of variable Intensity, the monochrome light sources being arranged in such a way as to produce the variable colour by synthesis of the specific colours of the monochrome light sources; and
   - an electronic control circuit (3) connected to the sensor (1) and configured to convert at least one measurement of the sensor into an electrical drive signal of the Indicator to cause the emission of a light of a resulting colour depending on the measurement of said physico-chemical parameter.

2. A colorimetric measurement and signalling device according to claim 1 in which the control circuit is adapted to produce a modulated pulse width signal for each monochrome light source so as to adjust the light intensity thereof.

3. A colorimetric measurement and signalling device according to one of claims 1 and 2 **characterised in that** it comprises a plurality of sensors (1A, 1B, 1C, ...) capable of respectively measuring distinct physico-chemical parameters of the medium to be analysed, the control circuit being respectively connected to each sensor and being capable of producing a distinct drive signal respectively for each of the light sources (2A, 2B, 2C) of the indicator (2) In response respectively to measurement of a distinct sensor (1A, 1B, 1C) in such a way that the light intensity emitted by each source is dependent respectively on the measurement of a sensor.

4. A colorimetric measurement and signalling device according to claim 1 and further comprising an electrolytic measurement chamber (7) and in which the sensor (1A, 1B, 1C, ...) comprises electrodes for being immersed in a flow of water passing through the electrolytic measurement chamber.

5. A colorimetric measurement and signalling device according to claim 1 comprising a housing (4) having a hollow body (4a) and a cover (4b) which define a sealed measuring chamber (7), at least a part of the housing being made of a transparent or translucent material, the sensor (1, 1A, 1B, 1C, ...) and the light indicator being accommodated in the housing (4).

6. A colorimetric measurement and signalling device according to claim 5 in which the cover (4b) of the housing is made of a transparent or translucent material and in which the light indicator (2, 2A, 2B, 2C, ...) is accommodated in the cover (4b) of the housing (4).

7. A colorimetric measurement and signalling device according to claim 5 in which the housing (4) is provided with an inlet opening (5) and an outlet opening (6) which are intended to permit the device to be connected to an outlet conduit (C) of a swimming pool so that when the housing (4) is fitted on said conduit the sensor (1; 1A, 1B, 1C) is immersed in a flow of water passing through the housing.

8. A colorimetric measurement and signalling device according to claim 1 comprising a sensor (10) permitting measurement of the strength of an electric electrolysis current and a temperature sensor (9).

9. A colorimetric measurement and signalling device according to claim 1 in which the light indicator (2) comprises at least one multicoloured light emitting diode (2').

10. A colorimetric measurement and signalling device according to claim 1 in which the monochrome sources (2A, 2B, 2C) comprise light emitting diodes (LED) of complementary colours.

11. A process for colorimetric measurement and display of physico-chemical values of a liquid medium **characterised in that** at least one sensor (1) sensitive to at least one physico-chemical parameter is immersed in the medium, at least one measurement signal from the sensor is passed to a control circuit (3) of a light indicator (2) capable of emitting light of a variable colour, and a control signal of the control circuit is passed to the light Indicator so as to emit a light whose colour is dependent on the measurement of the sensor (1), in which a light indicator (2) is used which has a plurality of monochrome light sources (2A, 2B, 2C) which are each capable of emitting a light of a specific colour and of a variable intensity, the monochrome light sources being arranged so as to produce said variable colour by synthesis of the specific colours.

12. A colorimetric measurement and display process according to claim 11 in which a plurality of distinct sensors (1A, 1B, 1C) is used, which are each designed to be sensitive to one of the physico-chemical parameters of the medium, and an electrical control signal is passed to each light source (2A, 2B, 2C) respectively in dependence on a measurement signal from each sensor, in such a way that each light source of the indicator (2) emits a light of an intensity which is dependent on the measurement of the corresponding sensor.

13. A process according to claim 11 in which the liquid medium Is water, the healthiness or sanitary state of which is to be analysed, and in which the light emitted by the light indicator is used to light the medium.

Fig. 1

Fig. 2

Fig. 3

Fig. 4

Fig. 5

Intensité lumineuse

Consigne PH

PH

**EP 2 966 438 B1**

## RÉFÉRENCES CITÉES DANS LA DESCRIPTION

**Documents brevets cités dans la description**

- US 5218304 A

- US 4033871 A